# EUROPÄISCHE PATENTSCHRIFT

(11) **EP 2 168 513 B1**
(45) Veröffentlichungstag und Bekanntmachung des Hinweises auf die Patenterteilung: **28.12.2011**
(21) Anmeldenummer: 08017211.7
(22) Anmeldetag: 30.09.2008
(51) Int. Cl.: A61B 17/80, A61B 17/86

(54) **Osteosyntheseplatte**
Bone plate
Plaque d'ostéosynthèse

(43) Veröffentlichungstag der Anmeldung: 31.03.2010
(73) Patentinhaber: Frowein EZH GmbH, 42117 Wuppertal (DE)
(72) Erfinder: Diez, Ingolf, 78532 Tuttlingen (DE)
(74) Vertreter: von Hellfeld, Axel

(56) Entgegenhaltungen:
- EP-A- 1 336 383
- EP-A- 1 878 398
- WO-A-00/69351
- WO-A-2006/103245
- WO-A-2007/009124

## Beschreibung

Die Erfindung betrifft eine Osteosynthesevorrichtung, die eine Platte aufweist mit mindestens einem Durchgangsloch, durch das eine Schraube in einem Knochen einschraubbar ist, zum Überbrücken von insbesondere Knochenfrakturen und zum Verbinden des Knochens mit der Platte, wobei die Platte um das Durchgangsloch eine Einlage aufweist aus einem im Vergleich zum Material der Platte weicheren Material, in die die Schraube zumindest teilweise selbst ein Gewinde schneidend einschraubbar ist.

Derartige Osteosyntheseplatten sind z. B. aus der DE 196 29 011 C2 bekannt. Auch die WO 2008/077482 A1 beschreibt eine Osteosynthesevorrichtung der hier in Rede stehenden Art. Weiteren Stand der Technik bilden die DE 195 45 612 T2, EP 1 272 114 B1, EP 1 570 796 B1, EP 1 088 522 B1, EP 1 143 867 B1, und die EP 1 211 994 B1. Dieser Stand der Technik wird nachfolgend als bekannt vorausgesetzt.

Derartige Osteosynthesevorrichtungen dienen also insbesondere dazu, die Lage von Knochenstücken bei Knochenfrakturen zu stabilisieren, um die Osteosynthese in der richtigen Relativlage der Fragmente zu fördern.

Der Stand der Technik gemäß der DE 196 29 011 C2 sieht bereits selbstschneidende Schrauben vor, die in eine im Verhältnis zur Platte weichere Einlage um die Durchgangslöcher herum eingeschraubt werden, insbesondere auch schräg in Bezug auf die Längsachse des Durchgangsloches.

Der Erfindung liegt die Aufgabe zugrunde, eine Osteosynthesevorrichtung der eingangs genannten Art bereitzustellen, die bei einfacher und kostengünstiger Herstellung eine hohe Funktionssicherheit im Einsatz währleistet. Das Dokument WO2007 009 124 A2 offenbart eine Platte gemäss dem Oberbegriff von Anspruch 1. Erreicht wird dies durch eine formschlüssige Verbindung zwischen der genannten Einlage und der Platte derart, dass die Einlage zumindest gegen Zugkraft zwischen der Platte und dem Knochen in ihrer Stellung in der Platte ortsfest gesichert (gehalten) ist.

Eine Osteosyntheseplatte der hier in Rede stehenden Art hat zwei Seiten: Die eine Seite ist dem Knochen zugewandt und die gegenüberliegende Seite der Platte ist somit vom Knochen abgewandt. Die Schraube wird auf der letztgenannten Seite der Platte, die man auch als Außenseite bezeichnen kann, in das Durchgangsloch der Platte eingeschraubt und ragt dann über die Innenseite der Platte hinaus und wird dabei in das Knochenfragment eingeschraubt. Wird die Platte also durch den Schraubvorgang in Richtung auf den Knochen gezogen, dann liegt eine "Zugkraft" im obigen Sinne vor und die Erfindung stellt durch die genannte formschlüssige Verbindung sicher, dass die Einlage, deren Material es ermöglicht, dass die Schraube selbstschneidend in der Einlage ihr Gewinde erzeugt, nicht aus der Platte in Richtung Knochen gezogen wird.

Die Erfindung sieht vor, dass die formschlüssige Verbindung auch in entgegengesetzter Richtung die Einlage stabil in der Platte positioniert, also aufgrund des Formschlusses die Einlage gegen Druckkräfte gesichert ist, d.h. Kräfte, welche die Einlage vom Knochen weg aus der Platte herausdrücken könnten.

Anders ausgedrückt, wirkt die formschlüssige Verbindung zwischen Einlage und Platte in beiden Richtungen parallel und antiparallel zur Längsachse des Durchgangsloches, d.h. praktisch in allen Richtungen.

Gemäß einer bevorzugten Ausgestaltung der Erfindung hat die formschlüssige Verbindung einen oder mehrere Vorsprünge am Umfang der Einlage, die in komplementäre Ausnehmungen in der Platte eingreifen. Entweder im Kombination mit der vorstehend genannten Ausgestaltung oder allein ohne diese, weist die Platte Vorsprünge auf.

Nach der Erfindung liegt die Einlage über ihre gesamte Mantelfläche satt an einer komplementären Fläche der Platte an. Es bestehen also keine Hohlräume zwischen der Einlage und der Platte.

Eine andere Ausgestaltung, die nicht unter die Ansprüche fällt, sieht vor, dass die formschlüssige Verbindung über eine Konusform von Einlage und Platte erreicht wird. Es kann auch vorgesehen sein, dass Einlage und Platte in Doppel-Konusform ausgeführt sind, derart, dass zwei Konusformen in entgegengesetzten Richtungen vorliegen, die sich etwa in der Mittelebene der Platte treffen, entweder derart, dass die Spitzen der Konusse einander zugekehrt sind oder voneinander weg weisen. Im ersten Fall entsteht ein Formschluss in Diabolo-Form und im zweiten Fall ein Formschluss in Rautenform.

Eine andere Ausgestaltung, die nicht unter die Ansprüche fällt und insbesondere eine einfache Herstellung der Osteosynthesevorrichtung ermöglicht, sieht vor, dass die Einlage zweiteilig ist.

Bevorzugt besteht die Platte der Osteosynthesevorrichtung aus einem Metall und die Einlage aus einem Material, das trotz hinreichender Formstabilität ermöglicht, dass bei Einschrauben einer Schraube diese sich selbstschneidend ihr Gewinde im Material der Einlage formt. Als hierfür besonders geeignetes Material hat sich PEEK (Polyetheretherketon) erwiesen. Geeignet sind aber auch andere Materialien, wie Kunststoffe mit den genannten Eigenschaften. Als Material für die Einlage sind insbesondere auch geeignet PE (Polyethylen) und PMMA (Polymethylmethacrylat), letzteres auch in Form von Knochenzement.

Der Begriff "Platte" im Zusammenhang mit Osteosynthesevorrichtungen ist ein Fachbegriff und erfasst insbesondere auch langgestreckte Gebilde, bei denen Durchgangslöcher nur in einer Reihe angeordnet sind.

Nachfolgend werden Ausführungsbeispiele der Erfindung anhand der Zeichnung näher beschrieben. Es zeigt:
Figur 1 schematisch einen Schnitt durch ein erstes Ausführungsbeispiel einer Platte für eine Osteosynthesevorrichtung im Schnitt im Bereich eines Durchgangsloches, dieses Ausführungsbeispiel fällt nicht unter die Ansprüche;
Figur 2 ein zweites Ausführungsbeispiel einer Platte für eine Osteosynthesevorrichtung schematisch im Schnitt im Bereich eines Durchgangstoches , dieses Ausführungsbeispiel fällt nicht unter die Ansprüche;
Figur 3 ein drittes Ausführungsbeispiel einer Platte für eine Osteosynthesevorrichtung im Schnitt im Bereich eines Durchgangsloches;
Figur 4 ein viertes Ausführungsbeispiel einer Platte für eine Osteosynthesevorrichtung schematisch im Schnitt im Bereich eines Durchgangstoches, dieses Ausführungsbeispiel fällt nicht unter die Ansprüche;
Figur 5 ein fünftes Ausführungsbeispiel einer Platte für eine Osteosynthesevorrichtung schematisch im Schnitt im Bereich eines Durchgangsloches, dieses Ausführungsbeispiel fällt nicht unter die Ansprüche; und
Fig. 6 und 7 ein sechstes Ausführungsbeispiel einer Platte für eine Osteosynthesevorrichtung schematisch im Schnitt im Bereich eines Durchgangsloches mit einer zweiteiligen Einlage, dieses Ausführungsbeispiel fällt nicht unter die Ansprüche.

Die Figuren zeigen jeweils Ausführungsbeispiele von Platten für eine Osteosynthesevorrichtung, wobei einander funktionsgleiche oder funktionsähnliche Komponenten und Merkmale mit gleichen Bezugsziffern, ggf. um Buchstaben ergänzt, versehen sind.

In den Figuren sind die Platten für die Osteosynthese nicht vollständig gezeigt, sondern nur diejenigen Bereiche der Platte, die erfoderdlich sind, um die Erfindung zu erläutern, also Plattenbereiche um ein Durchgangsloch herum, durch welches eine Schraube (nicht gezeigt) geführt werden kann. Es versteht sich, dass jede der genannten Platten in aller Regel eine Vielzahl von Durchgangslöchern aufweist, auch wenn die dargestellten Ausführungsbeispiele nur jeweils ein Durchgangsloch zeigen. Die anderen Durchgangslöcher sind dann entsprechend gestaltet.

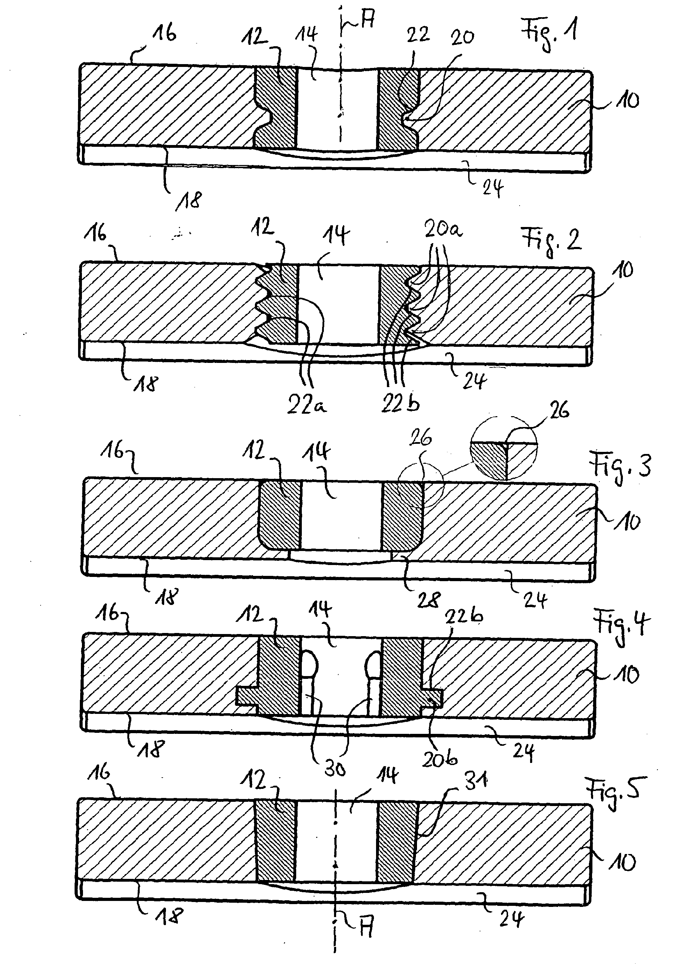

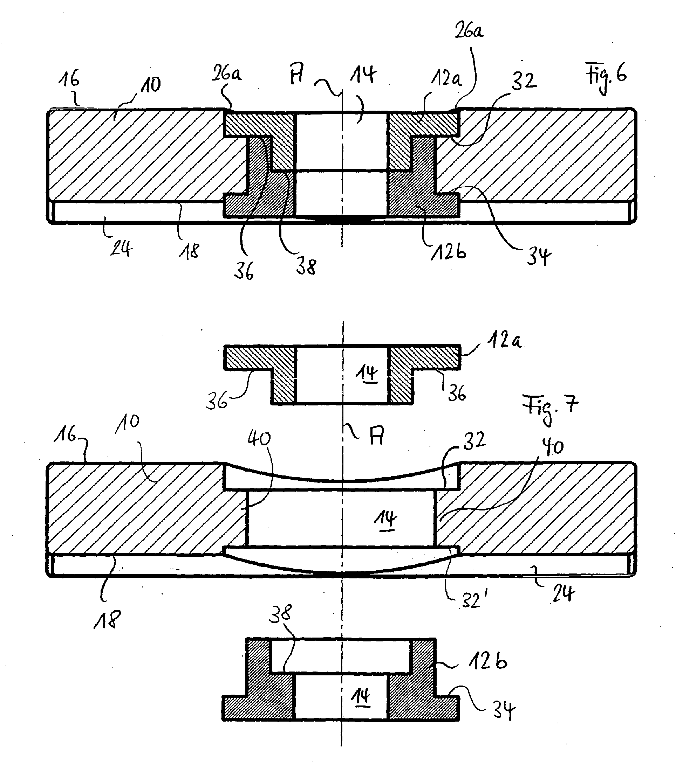

Beim ersten Ausführungsbeispiel einer Osteosyntheseplatte gemäß Figur 1 besteht die Platte 10 aus einem Metall, zum Beispiel Titan. In eine Öffnung in der Platte 10 ist eine Einlage 12 aus PEEK eingesetzt und durch Formschluss dort fest positioniert. In der Einlage 12 ist ein Durchgangsloch 14 ausgeformt, durch welches eine Schraube (nicht gezeigt) hindurch geschraubt wird, wobei die Schraube sich im Material der Einlage 12 ihr Gewinde selbst schneidet. Die Längsachse des Kreiszylinderförmigen Durchgangsloches 14 ist in den Figuren 1 und 5 sowie in Figur 6 eingezeichnet und verläuft bei den Beispielen gemäß den Figuren 2, 3 und 4 entsprechend. Die Schraube kann bei Einsatz der Platte 10 schräg, also in einem Winkel zur Längsachse A eingesetzt werden, je nach der chirurgischen Situation und der Lage der Knochenfragmente.

Die Platte 10 hat eine Außenseite 16 und eine Innenseite 18. Dies ist so zu verstehen, dass bei bestimmungsgemäßem Einsatz der Platte für eine Osteosynthese, die Innenseite 18 dem zu heilenden Knochen zugekehrt ist. In den Figuren greift also die Schraube von oben kommend durch das Durchgangsloch 14, so dass der Schraubenkopf im montierten Zustand der Platte oben liegt, d.h. auf der Außenseite 16.

Bei Ausführungsbeispiel gemäß Fig. 1 wird die formschlüssige Arretierung der Einlage 12 in der Platte 10 durch das Zusammenwirken eines Vorsprunges 20 in der Platte mit einer Vertiefung 22 in der Einlage 12 erzeugt. Vorsprung 20 und Vertiefung 22 können gemäß einer ersten Variante dieses Ausführungsbeispiels völlig rotationssymmetrisch sein, d.h. bei Drehung des in der Figur dargestellten Schnitts um die Achse A entsteht unter jedem beliebigen Drehwinkel dasselbe Bild. Gemäß einer abgewandelten Variante kann aber auch vorgesehen sein, dass als Verdrehsicherung für die Einlage 12 in Bezug auf die Platte 10 keine vollständige Rotationssymmetrie dergestalt von Einlage 12 und Platte 10 vorliegt. Bei dieser Variante kann z.B. die Gestalt des Außenumfanges der Einlage 12 (und entsprechend dann komplementär der Platte 10) in einem Schnitt senkrecht zur Längsachse A des Durchgangsloches 14 nicht kreisrund sein, so dass eine Drehung der Einlage 12 in Bezug auf die Platte 10 verhindert ist. Eine Verdrehsicherung im vorstehenden Sinne kann auch dadurch erreicht werden, dass die Einlage 10 an einer bestimmten Stelle ihres Umfanges einen Vorsprung (nicht gezeigt) aufweist, der sich nicht über den gesamten Umfang erstreckt, sondern nur an dieser Stelle in eine komplementäre Ausnehmung (nicht gezeigt) in der Platte 10 eingreift. Als Verdrehsicherung in diesem Sinne kommen auch mehrere, über den Umfang verteilte Vorsprünge in Betracht. Die beiden vorstehend genannten Varianten zur Erzeugung einer Verdrehsicherung zwischen Einlage 12 und Platte 10 lassen sich analog auf alle weiteren, unten näher beschriebenen Ausführungsbeispiele der Erfindung gemäß den Figuren 2, 3, 4, 5 und 6 anwenden.

Das Ausführungsbeispiel gemäß Figur 2 unterscheidet sich vom vorstehend beschriebenen Ausführungsbeispiel nach Figur 1 im wesentlichen dadurch, dass statt des einzigen Vorsprunges 20 und der einzigen Vertiefung 22 entsprechend Figur 1 bei Figur 2 mehrere Vorsprünge 20a in der Platte 10 ausgeformt sind, die in mehrere komplementäre Vertiefungen 22a in der Einlage 12 passgenau eingreifen. Ansonsten ergibt sich das Ausführungsbeispiel gemäß Figur 2 aus der vorstehenden Beschreibung von Figur 1.

Beim Ausführungsbeispiel gemäß Figur 3 wird die Einlage 12 auf der Innenseite 18 der Platte 10 über einen vorstehenden Rand 28 auf der Innenseite 18 gegen Zugkräfte im obigen Sinne formschlüssig gehalten, während die in der Regel weniger belastete formschlüssige Abstützung in der Gegenrichtung auf der Außenseite 16 der Platte 10 durch einen gebördelten Rand 26 der Platte 10 erreicht wird.

Das Ausführungsbeispiel gemäß Figur 4 zeigt eine Abwandlung des Ausführungsbeispiels nach Figur 1 derart, dass ein Vorsprung 20b an der Einlage 12 ausgebildet ist, der in eine Vertiefung 22b in der Platte 10 formschlüssig und passgenau eingreift. Weiterhin sind beim Ausführungsbeispiel nach Figur 4 in der Einlage 12 mehrere Schlitze 30 über den Umfang der Einlage 12 verteilt vorgesehen, die ermöglichen, dass beim Einbau der Einlage 12 in die Platte 10 die Einlage zusammengedrückt werden kann, damit der Vorsprung 20b in die Vertiefung 22b leicht einführbar ist.

Das Ausführungsbeispiel gemäß Fig. 4 bietet den besonderen Vorteil, dass eine Einlage in der Platte austauschbar ist, zum Beispiel bei einem Defekt der Einlage, und zwar vor Ort durch den Anwender.

Beim Ausführungsbeispiel nach Figur 5 wird die Arretierung der Einlage 12 durch Formschluss in der Platte 10 durch eine äußere Gestalt der Einlage 12 auf deren Mantelfläche in Form eines Konus 31 erreicht, der passgenau in einem entsprechenden komplementären Konus in der Platte 10 sitzt. Dies ermöglicht eine formschlüssige Sicherung der Einlage 12 in der Platte 10 gegen die oben definierten Zugkräfte. Soll die Einlage 12 auch durch Formschluss in entgegengesetzter Richtung (in Figur 5 also nach oben) in der Platte 10 gesichert werden, dann kann ein Doppel-Konus im oben beschriebenen Sinn eingesetzt werden, d.h. die äußere Kontur der Mantelfläche der Einlage 12 kann z.B. Diabolo-förmig sein und entsprechend die innere Kontur der an der Einlage 12 anliegenden Fläche der Platte etwas rautenförmig oder umgekehrt.

Die Figuren 6 und 7 zeigen ein weiteres Ausführungsbeispiel einer Osteosyntheseplatte mit einer Einlage, die hier aus zwei Elementen 12a, 12b besteht. Figur 6 zeigt die Platte mit eingebauter Einlage 12, während Figur 7 dieselbe Platte in Explosionsdarstellung (vor dem Zusammenbau) zeigt.

Wie in den Figuren 6 und 7 im einzelnen deutlich dargestellt ist, wird durch Stufen 32, 34, 36, 38 in den Komponenten 12a, 12b der Einlage und komplementären Stufen in der Platte 10 die formschlüssige feste Positionierung der beiden Einlagekomponenten in der Platte 10 bewirkt. Für den Formschluss ist in der Platte 10 ein Vorsprung 40 ausgeformt, der über Stufen 32, 32' mit den entsprechenden komplementären Stufen 34, 36 in den Einlagekomponenten 12a bzw. 12b zusammenwirkt. Auch bei diese Ausführungsbeispiel ist, wie bei allen anderen dargestellten Ausführungsbeispielen, der Formschluss über die Mantelfläche der Einlage lückenlos, d.h. es entstehen keine Hohlräume zwischen Einlage 12a, 12b und Platte 10.

Bei allen Ausführungsbeispielen kann wahlweise die Verbindung zwischen der Einlage 12 bzw. 12a, 12b und der Platte 10 durch Kleber gefördert werden.

Der bei den oben beschriebenen Ausführungsbeispielen gegebene Formschluss zwischen Einlage und Platte kann durch Kraftschluss, insbesondere Reibschluss, gefördert werden.

Auch kann bei allen Ausführungsbeispielen eine Verdrehsicherung in der oben anhand der Figur 1 näher beschriebenen Art vorgesehen sein.

## Patentansprüche

1. Osteosynthesevorrichtung, eine Platte (10) aufweisend mit mindestens einem Durchgangsloch (14), durch das eine Schraube in einem Knochen einschraubbar ist, zum Überbrücken von insbesondere Knochenfrakturen und zum Verbinden des Knochens mit der Platte (10), wobei die Platte um das Durchgangsloch (14) eine Einlage (12) aufweist aus einem im Vergleich zum Material der Platte (10) weicheren Material, in die die Schraube zumindest teilweise selbstschneidend einschraubbar ist,
mit einer formschlüssigen Verbindung (20, 22; 30; 32, 34, 36, 38) zwischen der Einlage (12; 12a, 12b) und der Platte (10) derart, dass die Einlage (12; 12a, 12b) zumindest gegen Zugkraft zwischen der Platte (10) und dem Knochen in der Platte (10) gesichert ist,
wobei die Platte (10) eine Innenseite (18) und eine Außenseite (16) aufweist, wobei die Innenseite (18) bei bestimmungsgemäßem Einsatz in der Osteosynthese dem zu heilenden Knochen zugekehrt ist,
wobei die Einlage (12) auf der Innenseite (18) der Platte (10) durch einen vorstehenden Rand(28) auf der Innenseite (18) gegen Zugkräfte formschlüssig gehalten wird,
**dadurch gekennzeichnet, dass** die Einlage (12) auf der Außenseite (16) der Platte (10) durch einen gebördelten Rand (26) der Platte in der Gegenrichtung formschlüssig abgestützt wird.

2. Osteosynthesevorrichtung gemäß Anspruch 1,
**dadurch gekennzeichnet, dass** die formschlüssige Verbindung (20, 22; 30; 32, 34, 36, 38) zwischen der Einlage (12; 12a, 12b) und der Platte (10) die Einlage auch gegen Druckkraft zwischen der Platte (10) und dem Knochen sichert.

3. Osteosynthesevorrichtung gemäß einem der Ansprüche 1 und 2,
**dadurch gekennzeichnet, dass** die formschlüssige Verbindung (20, 22; 30; 32, 34, 36, 38) einen oder mehrere Vorsprünge (12') am Umfang der Einlage (12) aufweist, die in eine bzw. mehrere komplementäre Vertiefungen (22b) in der Platte (10) eingreifen.

4. Osteosynthesevorrichtung nach einem der vorhergehenden Ansprüche,
**dadurch gekennzeichnet, dass** die formschlüssige Verbindung (20, 22; 30; 32, 34, 36, 38) einen oder mehrere Vorsprünge (20a; 28; 40) an der Platte (10) aufweist, die in eine bzw. mehrere Vertiefungen (22b) in der Einlage eingreifen.

5. Osteosynthesevorrichtung nach einem der vorhergehenden Ansprüche,
**dadurch gekennzeichnet, dass** die Einlage aus einem Kunststoff besteht, insbesondere PEEK (Polyetheretherketon).

6. Osteosynthesevorrichtung nach einem der vorhergehenden Ansprüche,
**dadurch gekennzeichnet, dass** die Platte aus einem Metall, insbesondere Titan, besteht.

7. Osteosynthesevorrichtung nach einem der vorhergehenden Ansprüche,
**dadurch gekennzeichnet, dass** die Einlage eine Verdrehsicherung aufweist derart, dass die Einlage (12) nicht um ihre Mittelachse (A) drehbar ist, insbesondere derart, dass die Einlage in einem Schnitt senkrecht zu ihrer Längsachse (A) nicht kreisförmig ist.

## Claims

1. An osteosynthesis device, comprising a plate (10) with at least one through hole (14) through which a screw may be screwed into a bone for bridging bone fractures and for joining the bone with the plate (10), wherein the plate comprises an insert (12) around the through hole (14), which insert comprisese a material which is softer than that of the plate (10), into which the insert the screw may be screwed while at least partially self-cutting a thread, wherein there is a positive locking (20, 22; 30; 32, 34, 36, 38) between the insert (12;12a, 12b) and the plate (10) such that the insert (12; 12a, 12b) is fixed at least against a pulling force between the plate (10) and the bone in the plate (10),
wherein the plate (10) comprises an inner side (18) and an outer side (16), the inner side (18) is directed towards the bone to be healed when using it for osteosynthesis,
wherein the insert (12), at the inner side (18) of the plate (10), is positively locked against pulling forces by a protruding rim (28),
**characterized in that** the insert (12) is positively locked against forces in the opposite direction at the outer side (16) of the plate (10) by means of a beaded edge (26).

2. The osteosynthesis device according to claim 1, **characterized in that** the positive locking (20, 22; 30; 32; 34, 36, 38) between the insert (12; 12a, 12b) and the plate (10) also secures the insert against compressive forces between the plate (10) and the bone.

3. The osteosynthesis device according to one of the claims 1 and 2, **characterized in that** the positive locking (20, 22; 30; 32, 34, 36, 38) comprises one or several protrusions (12') at the circumference of the insert (12) which engage in one or several, respectively, complementary recesses (22b) in the plate (10).

4. The osteosynthesis device according to one of the preceding claims, **characterized in that** the positive locking (20, 22; 30; 32, 34, 36, 38) comprises one or several protrusions (20a; 28; 40) at the plate (10), which engage in one or several, respectively, recesses (2b) in the insert.

5. The osteosynthesis device according to one of the preceding claims, **characterized in that** the insert consists of a synthetic material, in particular, of PEEK (polyetheretherketone).

6. The osteosynthesis device according to one of the preceding claims, **characterized in that** the plate consists of a metal, in particular, of titanium.

7. The osteosynthesis device according to one of the preceding claims, **characterized in that** the insert comprises an anti-rotation protection in such a manner that the insert (12) cannot be rotated about its centre axis (A), and in particular, in such a manner that the insert is not circular in a section perpendicular to tis longitudinal axis (A).

## Revendications

1. Dispositif d'ostéosynthèse, comprenant une plaque (10) présentant au moins un trou de passage (14) à travers lequel une vis peut être vissée dans un os, pour assurer le pontage notamment de fractures d'os et pour relier l'os à la plaque (10), la plaque présentant, autour du trou de passage (14), un insert (12) en un matériau plus mou comparé au matériau de la plaque (10), dans lequel la vis peut être vissée au moins en partie par auto-taraudage,
le dispositif d'ostéosynthèse comprenant une liaison par blocage mécanique (20, 22 ; 30 ; 32, 34, 36, 38) entre l'insert (12 ; 12a, 12b) et la plaque (10) de façon telle que l'insert (12 ; 12a, 12b) soit sécurisé dans la plaque (10) au moins à l'encontre d'une force de traction entre la plaque (10) et l'os,
la plaque (10) présentant un côté intérieur (18) et un côté extérieur (16), le côté intérieur (18) étant, en cas d'usage conforme au cours de l'ostéosynthèse, dirigé vers l'os à consolider,
l'insert (12) étant maintenu par blocage mécanique sur le côté intérieur (18) de la plaque (10), à l'encontre de forces de traction, par un bord en saillie (28) sur le côté intérieur (18),
**caractérisé en ce que** l'insert (12) est soutenu dans la direction opposée, par blocage mécanique, sur le côté extérieur (16) de la plaque (10), par un bord rabattu (26) de la plaque.

2. Dispositif d'ostéosynthèse selon la revendication 1,
**caractérisé en ce que** la liaison par blocage mécanique (20, 22 ; 30 ; 32, 34, 36, 38) entre l'insert (12 ; 12a, 12b) et la plaque (10) sécurise également l'insert à l'encontre d'une force de compression entre la plaque (10) et l'os.

3. Dispositif d'ostéosynthèse selon l'une des revendications 1 et 2,
**caractérisé en ce que** la liaison par blocage mécanique (20, 22 ; 30 ; 32, 34, 36, 38) présente une ou plusieurs protubérances (12') à la périphérie de l'insert (12), qui s'engagent dans un ou respectivement plusieurs creux complémentaires (22b) dans la plaque (10).

4. Dispositif d'ostéosynthèse selon l'une des revendications précédentes,
**caractérisé en ce que** la liaison par blocage mécanique (20, 22 ; 30 ; 32, 34, 36, 38) présente une ou plusieurs protubérances (20a ; 28 ; 40) sur la plaque (10), qui s'engagent dans un ou respectivement plusieurs creux (22b) dans l'insert.

5. Dispositif d'ostéosynthèse selon l'une des revendications précédentes,
**caractérisé en ce que** l'insert est réalisé en une matière plastique, notamment en PEEK (polyétheréthercétone).

6. Dispositif d'ostéosynthèse selon l'une des revendications précédentes,
**caractérisé en ce que** la plaque est réalisée en un métal, notamment en titane.

7. Dispositif d'ostéosynthèse selon l'une des revendications précédentes,
**caractérisé en ce que** l'insert présente un système anti-rotation tel que l'insert (12) ne puisse tourner autour de son axe central (A), notamment de telle manière que l'insert ne soit pas de forme circulaire dans une coupe perpendiculaire à son axe longitudinal (A).
